# EUROPEAN PATENT APPLICATION

(11) **EP 4 371 582 A1**
(43) Date of publication of application: **22.05.2024**
(21) Application number: 21950160.8
(22) Date of filing: 14.07.2021
(51) Int. Cl.: A61L 9/015, A61L 9/20

(54) **AIR PURIFIER AND PURIFICATION BOOTH FOR PREVENTING INFECTION**

(71) Applicant: International Frontier Technology Laboratory, Inc., Tokyo 105-0001 (JP)
(72) Inventor: KOMATSU, Nobuaki, Tokyo 105-0001 (JP); ITO, Tomoko, Tokyo 105-0001 (JP); WAKABAYASHI, Machiko, Tokyo 105-0001 (JP); NISHIYAMA, Takaya, Tokyo 105-0001 (JP); NAKAMURA, Haruyasu, Tokyo 105-0001 (JP); INOMATA, Tomoaki, Tokyo 105-0001 (JP); AIBA, Kazukiyo, Tokyo 105-0001 (JP)
(74) Representative: Ter Meer Steinmeister & Partner
(86) International application number: PCT/JP2021/026514
(87) International publication number: WO 2023/286219

(57) **Abstract**

Aspects relate to providing an air purifier and a purification booth for preventing infections that can effectively suppress ozone generation while sanitizing the air using relatively short-wavelength ultraviolet light. The air purifier includes: a housing 10 in which a reflux chamber CC is formed between an intake opening 11 and an exhaust opening 12; an ultraviolet lamp 23 that simultaneously generates ultraviolet rays with a wavelength of 185 nm and ultraviolet rays with a wavelength of 254 nm inside the reflux chamber CC; a manganese dioxide-based catalytic filter 25 through which air passes while proceeding from the reflux chamber CC toward the exhaust opening 12; and a reflux plate 24 which is disposed inside the reflux chamber CC and which is hit by a portion of the air flowing from the intake opening 11 toward the exhaust opening 12, thereby causing the portion of air to reflux, wherein the ultraviolet rays emitted by the ultraviolet lamp 23 irradiate both the air proceeding from the intake opening 11 to the reflux plate 24 and the portion of air flowing back from the reflux plate 24.

## Description

### [Technical Field]

The present invention relates to an air purifier and a purification booth for preventing infection using the same.

### [Background of the Invention]

The recent spread of the novel coronavirus infection (COVID-19) has caused severe damage to society as a whole, and the consideration of countermeasures is urgently required. As the main route of infection of the novel coronavirus infection is believed to be droplet infection (droplets from sneezes and coughs), in order to prevent the spread of infection, it is extremely important to sterilize the viruses contained in droplets to prevent their spread. At the same time, with respect to droplet infection, as it was found that virus droplets can survive in the air for more than three hours, infections closer to airborne infections are increasingly likely.

In particular, it is an urgent issue to take measures to prevent the spread of infection to medical workers and other related individuals, especially in medical settings where infected people receive treatment and hotels where infected persons are staying.

In order to remove droplets scattered in the air, HEPA filters can remove over 99.97% of particles up to 0.3µ in size. By additionally using an electrostatic filter for even smaller particles, it is possible to achieve a significant sterilization effect. On the other hand, if the size of a virus is 0.1µ or less, even if it is captured by a filter, it can survive for a considerable time. For this reason, deep ultraviolet light with a wavelength of 185 nm is used to generate ozone, which sterilizes droplet nuclei as well. Next, ultraviolet rays of 200 nm to 280 nm destroy the constituent bases of DNA and RNA types, making it possible to perform sterilization in a short time. Accordingly, one type of conventional air purifier uses ozone, ultraviolet light, or a combination thereof to sterilize the air, which is then filtered and released. Each irradiation wavelength has its own characteristics, but generally speaking, the shorter the wavelength, the more energy it takes to destroy genes. In particular, light with a wavelength of 185 nm is effective in destroying viruses and bacteria in a few seconds.

It is known that DNA and RNA, which are nucleic acids that form the main body of the genes of viruses and bacteria, exhibit the highest absorption characteristics at a wavelength of around 260 nm. Low-pressure mercury lamps have a high emission spectrum near a wavelength of 254 nm. Utilizing these properties, when the target is bacteria, irradiation from a low-pressure mercury lamp destroys the cell membrane of the bacteria and drains the body fluid, killing the target, and when the target is a virus, the genetic information in the DNA and RNA of the virus is broken down to prevent its proliferation.

However, it is also known that there is a risk that irradiating the human body with ultraviolet rays in this wavelength range may have an adverse effect on the human body. The skin is divided into four layers, namely, the stratum corneum, the stratum granulosum, the stratum spinosa, and the stratum basale, in order from the layer nearest the surface. When the human body is irradiated with ultraviolet rays having a wavelength of 254 nm, the ultraviolet rays penetrate the stratum corneum, reach the stratum granulosum, the stratum spinosa, and possibly the stratum basale, and are absorbed by the DNA of the cells present in these strata. This results in DNA damage and increases the risk of skin cancers. On the other hand, it has been reported that ultraviolet rays of 222 nm damages the DNA of viruses and bacteria, but does not reach the nucleus of the human body and does not cause DNA damage. Accordingly, Patent Document 1 discloses a technique for disinfecting a human body by irradiating the human body with ultraviolet rays having a wavelength of greater than or equal to 200 nm and less than or equal to 230 nm.

Although short-wavelength ultraviolet rays have the ability to sterilize, they also react with oxygen in the air and produce ozone, which is harmful to the human body. Active oxygen is generated by the reaction between ozone and ultraviolet rays with a wavelength of 100 to 260 nm, and its strong oxidizing action decomposes contaminated organic material. Ozone has a strong oxidizing power that is several times that of chlorine. This strong oxidizing power causes the deterioration of most substances.

Ozone is strongly capable of oxidizing substances, second only to fluorine and several times stronger than chlorine, and is used for sterilization, disinfection, deodorization and the like. However, due to this oxidizing power, high concentrations of ozone cause the deterioration of materials such as rubber, plastics, and the like, and also damage living organisms. Ozone is not easily absorbed by water, so if it is taken into the respiratory system, it may reach deep parts of the lungs and cause respiratory disorders (such as pulmonary edema). With respect to this, Patent Document 2 discloses a technique for decomposing ozone generated by using ultraviolet rays.

### [Citation List]

### [Patent Documents]

[Patent Document 1] Japanese Patent No. 6493703 B
[Patent Document 2] Japanese Patent Application No. H9-52020

### [Summary of Invention]

### [Technical Problem]

In the sterilization technique described in Patent Document 1, the wavelength of the ultraviolet rays used is greater than or equal to 200 nm, and short wavelength ultraviolet rays in the 185 nm wavelength range are excluded. Further, the ozone decomposition technique of Patent Document 2 is mainly directed toward improving deodorization performance, and there is room for further improvement in order to use it in an air purifier that provides sterilized air, for example.

The present invention has been made in view of the above problems, and an object thereof is to provide an air purifier and a purification booth for preventing infection that can effectively suppress the generation of ozone while sterilizing air using ultraviolet rays with a relatively short wavelength.

### [Means for Solving the Problems]

In order to solve the above problems, the air purifier of the present invention includes a housing in which a reflux chamber is formed between an intake opening and an exhaust opening; an ultraviolet irradiation device that simultaneously generates ultraviolet rays with a wavelength of 185 nm and ultraviolet rays with a wavelength of 254 nm inside the reflux chamber; an ozone decomposition member through which air passes while proceeding from the reflux chamber toward the exhaust opening; and a reflux plate which is disposed inside the reflux chamber, is hit by a portion of the air flowing from the intake opening toward the exhaust opening, and causes the portion of the air to reflux; wherein: the ultraviolet rays emitted by the ultraviolet irradiation device irradiate both the air proceeding from the intake opening to the reflux plate and the portion of the air flowing back from the reflux plate.

### [Advantageous Effects of Invention]

According to the present invention, it is possible to provide an air purifier and a purification booth for preventing infection that can effectively suppress the generation of ozone while sterilizing air using ultraviolet rays with a relatively short wavelength.

### [Brief Description of Drawings]

[Fig. 1] FIG. 1 is a perspective view of an air purifier according to a first embodiment of the present invention, viewed obliquely from above, in which air flows are indicated by arrows.
[Fig. 2] FIG. 2 is a longitudinal cross-sectional view of an air purifier according to a second embodiment of the present invention, in which air flows are indicated by arrows.
[Fig. 3] FIG. 3 is a diagram illustrating a purification booth for preventing infection in which the air purifier according to the embodiments of the present invention is used in combination as the intake device and exhaust device of an isolation booth.
[Fig. 4] FIG. 4 is a longitudinal cross-sectional view of the air purifier according to the second embodiment, in which air flows are indicated by arrows.

### [Description of Embodiment(s)]

Hereinafter, the embodiments will be described with reference to the drawings. The air purifier and the purification booth for preventing infection according to the present invention are not limited to the following embodiments.

### <Air Purifier>

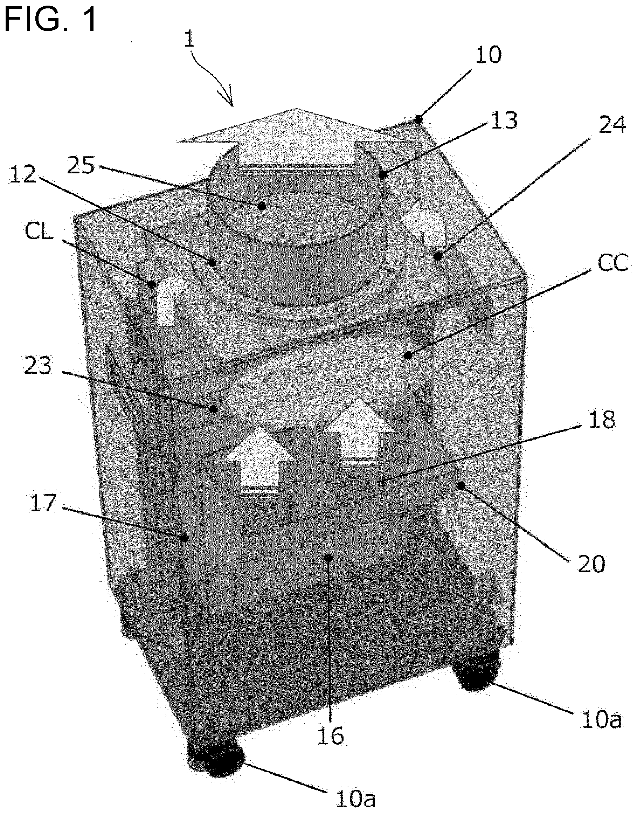

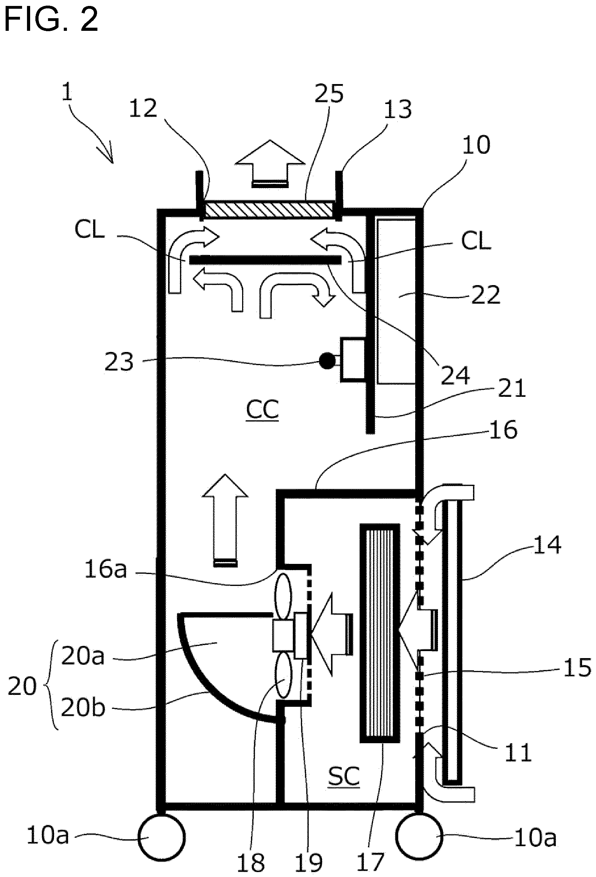

FIG. 1 and FIG. 2 are a schematic perspective view and a longitudinal cross-sectional view illustrating the air purifier 1 of the first embodiment, respectively. In the figures, a housing 10 supported on the lower surface by casters 10a is provided with an intake opening 11 on its rear surface and an exhaust opening 12 on the upper surface. The periphery of the exhaust opening 12 is surrounded by an exhaust pipe 13, and a manganese dioxide-based catalytic filter 25 is disposed inside the exhaust opening 12 as an ozone decomposing member.

A shielding plate 14 is disposed outside the intake opening 11 with respect to the intake opening 11 to prevent relatively large foreign objects from being sucked in. Furthermore, the intake opening 11 is covered with a pre-filter 15 having a mesh.

Here, as the material of the pre-filter 15, a textile product such as polypropylene, polyvinylidene chloride, polyester, or nylon can be used. The purpose of installing the pre-filter 15 is to first collect coarse particles suspended in the air.

The inside of the housing 10 is partitioned into an intake opening 11 side and an exhaust opening 12 side by a partition plate 16. The chamber on the intake opening 11 side partitioned by the partition plate 16 is defined as the intake chamber SC, and the chamber on the exhaust opening 12 side partitioned by the partition plate 16 is defined as the reflux chamber CC. The volume of the reflux chamber CC is greater than the volume of the intake chamber SC. The partition plate 16 is formed with a communication hole 16a that allows the intake chamber SC to communicate with the reflux chamber CC.

In the intake chamber SC, a HEPA filter 17 is disposed close to the intake opening 11, and the air entering through the intake opening 11 can pass therethrough.

When installing the pre-filter 15 and the HEPA filter 17, ethylene propylene diene rubber, silicone rubber, fluororubber, or the like can be used as a packing to prevent air leakage.

Further, a fan 18 driven by a motor 19 is disposed in the intake chamber SC close to the communication hole 16a of the partition plate 16. The motor 19 is connected to a power supply (not illustrated in the figures). Various types such as an axial flow type or a centrifugal type can be used for the fan 18.

In the reflux chamber CC, a rectifier plate 20 is attached to the partition plate 16 so as to surround the communication hole 16a. The rectifier plate 20 is formed, for example, by press-forming a metal plate, for example, and includes a pair of fan-shaped side plates 20a extending from both sides of the communication hole 16a, and a curved plate 20b connecting the fan-shaped side plates 20a. The upper portion of the rectifier plate 20 is open.

It should be noted that the rectifier plate 20 does not necessarily have to be provided so as to surround the communication hole 16a, and instead of the configuration with the fan-shaped side plates 20a and the curved plate 20b, a simple plate-shaped rectifier plate may be provided at a position on the curved plate 20b so that the air can be deflected upward, for example.

Further, if it is possible to cause air to convect in the reflux chamber CC, the rectifier plate 20 need not be provided.

In the reflux chamber CC, a holding plate 21 extends downward from the upper wall of the housing 10, and a control circuit 22 is disposed between the holding plate 21 and the rear surface of the housing 10. The control circuit 22 is connected to a power source (not illustrated in the figures), and is capable of driving and controlling the ultraviolet lamp (the ultraviolet irradiation device) 23 exposed to the reflux chamber CC side of the holding plate 21 to emit light. The ultraviolet lamp 23 can simultaneously emit light at a wavelength of 185 nm and a wavelength of 254 nm.

A reflux plate 24 is arranged between the ultraviolet lamp 23 and the exhaust opening 12 so as to be spaced apart from the upper wall of the housing 10 and extend parallel to the upper wall. A gap CL is formed between the reflux plate 24 and the inner circumference of the housing 10. Here, it is preferable that the reflux plate 24 covers the entire exhaust opening 12 when viewed in the axial direction of the exhaust opening 12 (the vertical direction in FIG. 2).

Next, the operation of the air purifier 1 of the present embodiment will be described. The air purifier 1 of the present embodiment is used by being placed indoors, for example, but there are no particular restrictions on the usage environment.

By turning on a power switch (not illustrated in the figures), power is supplied to the motor 19, the fan 18 rotates, power is supplied to the control circuit 22, and the ultraviolet lamp 23 emits light.

As the fan 18 rotates, as indicated by the arrow in FIG. 2, air or outside air containing viruses, bacteria, and various contaminants is sucked into the intake chamber SC through the pre-filter 15 of the intake opening 11, and further passes through the HEPA filter 17, such that particles of 0.3 µm are collected with a probability of 99.9% or more.

According to the guidelines for isolation precautions (Guideline for Isolation Precautions : Preventing Transmission of Infectious Agents in Healthcare Settings 2007) provided by the United States Center for Disease Control and Prevention (CDC), since the size of droplet nuclei containing infectious microorganisms that cause airborne infections such as the influenza virus, tuberculosis bacteria, and measles virus are usually less than 5 µm, a considerable amount of contaminants are collected by passing through the HEPA filter 17.

The air taken into the intake chamber SC enters the reflux chamber CC through the communication hole 16a of the partition plate 16, and flows toward the reflux plate 24 above. At this time, the air flowing from the communication hole 16a enters the reflux chamber CC having a relatively large capacity, thereby reducing its flow velocity, passes through the vicinity of the ultraviolet lamp 23 and reaches the reflux plate 24. At this time, the aerosols and droplet nuclei of contaminants that were not collected by the HEPA filter 17 are damaged and destroyed at the genetic level within a few seconds by the ultraviolet rays irradiated from the ultraviolet lamp 23 (see Shoichi Morimoto "Controlling airflow to reduce airborne infection risks," Clinical Ecology, Vol. 27, No. 1 (2018), Hiroshi Ida, Satoko Haneda, U Yanagi, Syunsuke Sejima, Yoshio Nakanishi "Infection control of an air-conditioning system incorporating UVGI," Clinical Ecology, Vol. 27, No. 1 (2018).

A portion of the air that passed through the vicinity of the ultraviolet lamp 23 passes through the reflux plate 24, the inner periphery of the housing 10, and the gap CL toward the exhaust opening 12, but most of the air hits the reflux plate 24 and is refluxed, such that the flow is changed toward the partition plate 16 within the reflux chamber CC. The air refluxed at this time passes near the ultraviolet lamp 23 again, so that the aerosols and the droplet nuclei are further destroyed by the irradiated ultraviolet rays. Furthermore, since the air flowing toward the partition plate 16 flows toward the reflux plate 24 together with the air newly introduced into the reflux chamber CC from the communication hole 16a, it is irradiated with ultraviolet rays from the ultraviolet lamp 23. In this way, according to the present embodiment, by refluxing a portion of the air in the reflux chamber CC, it can be irradiated by ultraviolet rays for a longer period of time, thereby increasing the effect of detoxifying contaminants. It should be noted that since the ultraviolet lamp 23 is mounted in a sealed manner within the housing 10, ultraviolet rays will not leak and come into contact with human eyes.

Incidentally, the ultraviolet rays emitted from the ultraviolet lamp 23 with a wavelength of 185 nm contribute to the generation of ozone. More specifically, when an oxygen molecule absorbs photons having a wavelength 185 nm, it decomposes into oxygen atoms (active oxygen) as illustrated in the following Equation 1, and the oxygen atoms combine with the oxygen molecules to generate ozone.

(Equation 1) 3O₂ + hv → [active oxygen such as O or O₂ ⁻] → 2O₃

Even in a completely sealed environment at room temperature, ozone decreases by more than half in a day, and is eventually reduced to its original oxygen. However, when the human body inhales air containing even a small amount of ozone, even for a short period of time, ozone comes into contact with the nasal cavity, throat, trachea, lungs, and the like, and the surface becomes oxidized, which can cause symptoms such as odor, irritation, coughing, headaches, drowsiness, and chest tightness. In order to prevent damage caused by the toxicity of ozone, in Japan, the Tolerable Concentration Committee of the Japanese Society of Industrial Hygiene has set the allowable concentration of ozone in the working environment to 0.1 ppm.

As described above, by irradiating the air introduced into the reflux chamber CC with ultraviolet rays for a relatively long period of time, a detoxifying effect on contaminants can be expected, but on the other hand, the amount of ozone generated also increases. Therefore, the air purifier 1 of the present embodiment includes a manganese dioxide-based catalytic filter 25. More specifically, the ozone-containing air that passes through the reflux plate 24, the inner periphery of the housing 10, and the gap CL toward the exhaust opening 12 passes through the manganese dioxide-based catalytic filter 25, such that the ozone is decomposed.

Activated carbon has traditionally been used as a simple method for removing ozone. Ozone is adsorbed and reacts within the pores of activated carbon, and is decomposed into carbon dioxide and water. Although activated carbon is relatively easy to manage, there is a problem in that it is difficult to use because it causes an exothermic reaction as shown in Equation 2 below.

(Equation 2) C (activated carbon) + 2O₃ → CO₂ + 2O₂ + 679kJ

With respect to this, composite oxide catalysts, such as manganese dioxide catalysts, can lower the activation energy of the ozone auto-decomposition reaction and convert ozone into oxygen even at room temperature or low temperature as shown in Equations 3 and 4, making it harmless.

(Equation 3) O₃ + X (catalytic) → X-O + O₂

(Equation 4) X-O + O₃ → X + 2O₂

That is, composite oxide catalysts have a higher ozone decomposition efficiency than activated carbon, and can maintain their performance for a long period of time. The feature of these catalysts is that the ozone adsorption → decomposition → desorption reaction occurs rapidly at room temperature, allowing it to continue exhibiting higher decomposition performance for a long time. The manganese dioxide-based catalytic filter 25 can be made by sintering powder into pellets, as a honeycomb-shaped molded product, a honeycomb-shaped material, or supported on a fibrous base material.

### <Other Embodiments of the Air Purifier>

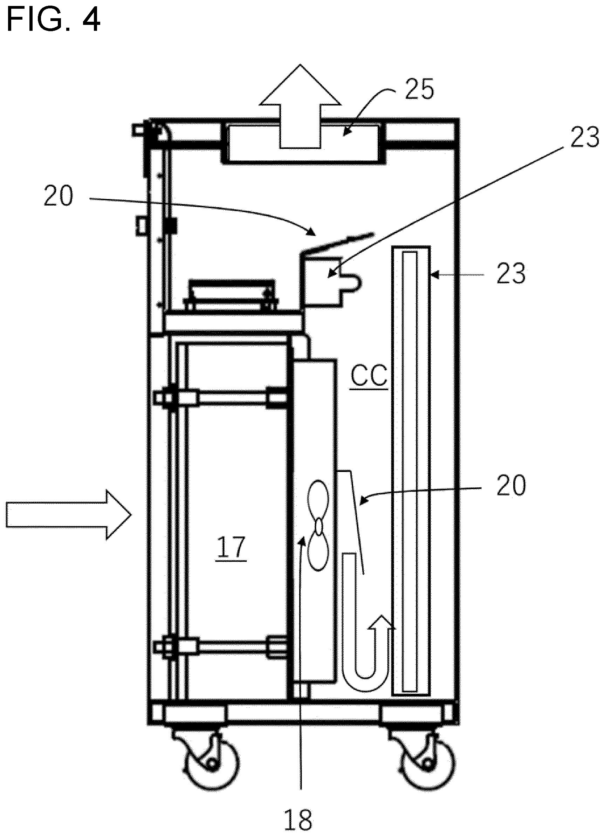

FIG. 4 is a cross-sectional view of the air purifier according to a second embodiment. In the second embodiment, as illustrated in FIG. 4, by installing an ultraviolet lamp 23 vertically along the flow path of the air in the device, it is possible to increase the irradiation time of the ultraviolet rays with respect to the air, and enhance the sterilization effect.

Additionally, the ultraviolet lamp 23 installed horizontally as illustrated in the first embodiment may be used in combination with the vertical ultraviolet lamp 23, or need not be used. When an ultraviolet lamp installed horizontally and an ultraviolet lamp installed vertically are used in combination, the sterilization effect can be further enhanced.

For example, when ultraviolet rays having a wavelength of 185 nm are generated from the horizontally installed ultraviolet lamp 23, ultraviolet rays having a wavelength of 254 nm can be generated from the vertically installed ultraviolet lamp 23, thereby enhancing the sterilization effect.

That is, both ultraviolet rays having a wavelength of 185 nm and ultraviolet rays having a wavelength of 254 nm may be generated from both of the horizontally installed ultraviolet lamp and the vertically installed ultraviolet lamp, or the generated wavelengths may be appropriately combined and generated by the horizontally installed ultraviolet lamp and the vertically installed ultraviolet lamp.

In addition, the number of the ultraviolet lamps installed vertically may be one, or a plurality of the ultraviolet lamps may be arranged in parallel.

Further, in the second embodiment, by attaching the rectifier plate 20 facing downward, it is possible to efficiently generate air convection without using the reflux plate 24 employed in the first embodiment, and it becomes possible to maximize the use of the ultraviolet lamp.

### <Reflective Member>

In order to effectively utilize the ultraviolet rays generated from the ultraviolet lamp inside the air purifier, a reflective member may be provided inside the housing. By providing the reflective member, the ultraviolet rays generated from the ultraviolet lamp are repeatedly reflected within the device, thereby further enhancing the air sterilization effect.

It should be noted that the reflective member need not be an independent member used only for reflection, and may be installed by forming a reflective film on the inner wall of the housing or the surface of the rectifier plate or the like.

### <Purification Booth for Preventing Infection>

In order to use the above-described air purifier 1 more effectively in the medical field, for example, the air purifier 1 according to the present embodiments can be used as an air supply device or an exhaust device of a simple booth 30 forming a certain space for isolating an infected person, for example.

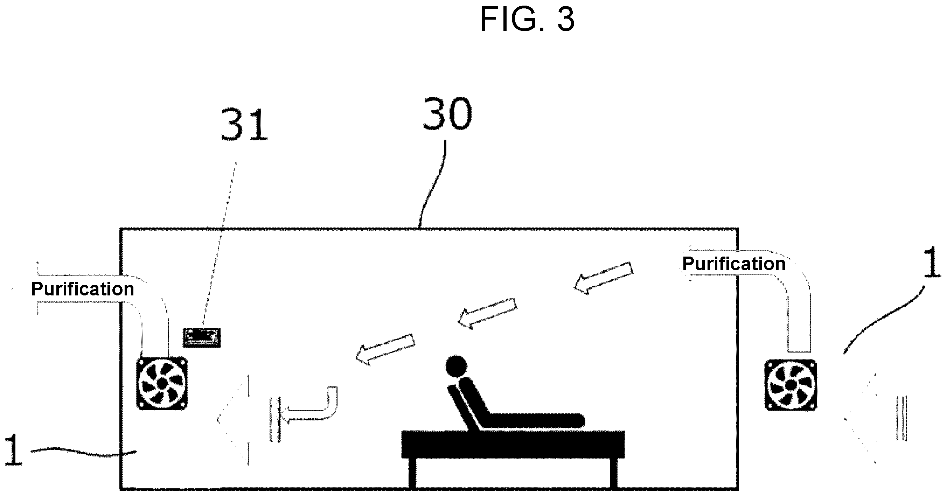

As illustrated in FIG. 3, when the above-described air purifier 1 is combined as an air supply device with a simple booth 30, the harmful components present in the outside air are decomposed by irradiating ultraviolet rays with an ultraviolet lamp 23 that simultaneously emits wavelengths of 185 nm and 254 nm. The ozone generated by the ultraviolet lamp 23 is converted to oxygen by the manganese dioxide-based catalytic filter 25, and by sending this detoxified air into the simple booth 30, the specific space where an infected person resides can be effectively cleaned.

Further, in the case that the above-described air purifier 1 is combined as an exhaust device with the simple booth 30, the air discharged into the atmosphere from inside the simple booth 30 can be purified by a similar action. By doing so, it becomes possible to detoxify the viruses in the air flowing out from inside the simple booth 30 and then exhaust it into the atmosphere.

Furthermore, if a similar air purifier 1 is installed as an intake device and an exhaust device of the simple booth 30 in a so-called push-pull type arrangement, the cleaning in the simple booth 30 can be made even more effective. In this case, it is desirable that the air introduced into the simple booth 30 from the air purifier 1 serving as the intake device flows along the diagonal of the simple booth 30 and is taken into and exhausted from the air purifier 1 serving as the exhaust device.

Further, in preparation for cases in which troubles such as clogging or the like occur in the manganese dioxide-based catalytic filter 25 of the air purifier 1, it is desirable to install an ozone sensor 31 in the simple booth 30 that detects that the ozone concentration has become 0.1 ppm or higher and issues an alarm.

According to the present embodiment, the short-wavelength ultraviolet rays can cleanse harmful components present indoors (inside the simple booth), and the ozone generated by the ultraviolet rays, which is harmful to the human body, can be made harmless. The push-pull arrangement creates a unidirectional flow inside the room (inside the simple booth), making it possible to efficiently remove harmful components. This configuration also has the advantage of not exposing the human body to harmful ultraviolet rays and ozone.

### (Examples)

Hereinafter, the results of experiments conducted by the present inventors will be explained.

Using the air purifier 1 of the present embodiment, when the amount of ozone generated by irradiating the air in the reflux chamber CC with ultraviolet rays from an ultraviolet lamp was measured using an ozone detector (ozone checker) OC-300 manufactured by Sato Shoji Co., Ltd. ozone of 0.25 ppm or more was detected, exceeding the measurement range. However, when the amount of ozone in the air that passed through the manganese dioxide-based catalytic filter 25 was measured using the same ozone detector, the amount of ozone was 0.000 ppm or less.

Further, when the particle count inside and outside the simple booth 30 was measured using a RION Handheld Particle Counter KC-51 manufactured by Rion Co., Ltd after the air purifier 1 of the present embodiment was installed in the simple booth 30 and operated for 60 minutes, the measurements illustrated in the following Table 1 were obtained. It should be noted that the simple booth 30 was installed in a typical office. Further, in the arrangement example illustrated in FIG. 3, it was confirmed by an anemometer that the air volume on the intake side of the air purifier 1 in the simple booth 30 was greater than or equal to 135m³/hr.

**[Table 1]**

| Particle Count Inside and Outside the Booth Units: Counts/ft³ | | | |
|---|---|---|---|
| Particle Diameter | Inside Booth | Outside Booth | Outside Environment (Reference) |
| 0.3 µm or more | 7,995 | 40,615 | 127,251 |
| 0.5 µm or more | 513 | 2,734 | 14,433 |
| 5.0 µm or more | 6 | 6 | 354 |

### [Reference Signs List]

- 1: Air purifier
- 10: Housing
- 11: Intake opening
- 12: Exhaust opening
- 13: Exhaust pipe
- 14: Shielding plate
- 15: Pre-filter
- 16: Partition plate
- 17: HEPA filter
- 18: Fan
- 19: Motor
- 20: Rectifier plate
- 21: Holding plate
- 22: Control circuit
- 23: Ultraviolet lamp
- 24: Reflux plate
- 25: Manganese dioxide-based catalytic filter
- 30: Simple booth
- 31: Ozone sensor
- CC: Reflux chamber
- SC: Intake chamber
- CL: Gap

## Claims

1. An air purifier comprising:
a housing in which a reflux chamber is formed between an intake opening and an exhaust opening;
an ultraviolet irradiation device that simultaneously generates ultraviolet rays with a wavelength of 185 nm and ultraviolet rays with a wavelength of 254 nm inside the reflux chamber;
an ozone decomposition member through which air passes while proceeding from the reflux chamber toward the exhaust opening; and
a reflux plate which is disposed inside the reflux chamber, is hit by a portion of the air flowing from the intake opening toward the exhaust opening, and causes the portion of the air to reflux;
wherein:
the ultraviolet rays emitted by the ultraviolet irradiation device irradiate both the air proceeding from the intake opening to the reflux plate and the portion of the air flowing back from the reflux plate.

2. An air purifier comprising:
a housing in which a reflux chamber is formed between an intake opening and an exhaust opening;
an ultraviolet irradiation device that simultaneously generates ultraviolet rays with a wavelength of 185 nm and ultraviolet rays with a wavelength of 254 nm inside the reflux chamber; and
an ozone decomposition member through which air passes while proceeding from the reflux chamber toward the exhaust opening;
wherein:
the ultraviolet irradiation device is installed in the vertical direction of the housing; and
the ultraviolet rays emitted by the ultraviolet irradiation device irradiate the air proceeding from the intake opening toward the exhaust opening.

3. The air purifier according to claim 2, wherein:
an ultraviolet irradiation device is installed in both the horizontal direction of the housing and the vertical direction of the housing;
the ultraviolet irradiation device installed in the horizontal direction of the housing generates ultraviolet rays with a wavelength of at least 185 nm; and
the ultraviolet irradiation device installed in the vertical direction of the housing generates ultraviolet rays with a wavelength of at least 254 nm.

4. The air purifier according to any one of claim 1 to 3, further comprising:
a partition that divides the inside of the housing into the reflux chamber on an exhaust opening side and an intake chamber on an intake opening side;
wherein:
a HEPA filter through which air that enters through the intake opening passes is disposed in the intake chamber.

5. The air purifier according to claim 4, further comprising:
a rectifier plate that directs a flow of air that has entered the reflux chamber from the intake chamber toward the exhaust opening side.

6. The air purifier according to any one of claims 1 to 5, wherein:
the ozone decomposition member is a manganese dioxide-based catalytic filter.

7. The air purifier according to any one of claims 1 to 6, wherein:
the air purifier includes a reflective member in the housing.

8. The air purifier according to claim 7, wherein:
the reflective member is a reflective film formed on a surface of a part of a member in the housing.

9. A purification booth for preventing infection **characterized in that** the air purifier according to any one of claims 1 to 8 is installed as an air supply device or an exhaust device in a booth forming a certain space.
